# EUROPEAN PATENT APPLICATION

(11) **EP 3 133 111 A1**
(43) Date of publication of application: **22.02.2017**
(21) Application number: 15020140.8
(22) Date of filing: 21.08.2015
(51) Int. Cl.: C08K 3/00, C08K 3/08, C08L 83/04, C11D 3/48

(54) **METHOD FOR FABRICATION OF SILVER NANOPARTICLES CONTAINING ALIPHATIC SILICONE ACRYLATE BASED ORGANIC-INORGANIC COMPOSITE COATING WITH ANTIBACTERIAL ACTIVITY**

(71) Applicant: Kaunas University of Technology, 44249 Kaunas (LT)
(72) Inventor: Jankauskaité, Virginija, LT-51424 Kaunas (LT); Lazauskas, Algirdas, LT-51423 Kaunas (LT); Baltrusaitis, Valentinas, LT-51424 Kaunas (LT)

(57) **Abstract**

The present invention provides a practical method of fabricating silver nanoparticles containing aliphatic silicone acrylate based UV-cured organic-inorganic composite coating with antibacterial activity. The present invention relates to a novel organic-inorganic hybrid formulation and synthesis method where cross-linking of aliphatic silicone acrylate based organic phase is obtained simultaneously with photochemical formation of silver nanoparticles via direct photoreduction of silver salt by UV-radiation. The organic-inorganic hybrid formulation contains aliphatic silicone acrylate based UV-curable polymer, silver salt, UV photoinitiator and organic solvent.

## Description

### Field of the Invention

The present invention relates to a photochemical synthesis method for fabrication of silver nanoparticles containing aliphatic silicone acrylate based UV-cured organic-inorganic composite coating with antibacterial activity. In particular, the present invention relates to a novel organic-inorganic hybrid formulation and synthesis method where cross-linking of aliphatic silicone acrylate based organic phase is obtained simultaneously with photochemical formation of silver nanoparticles via direct photoreduction of silver salt by UV-radiation.

### Background of the Invention

Organic-inorganic composite materials have gained increased interest due to their unique multifunctional properties. Different combinations of organic-inorganic materials thereof allow achieving exceptional optical, mechanical, thermal, electrical, catalytic, antibacterial and radiation-resistant properties with potential applications covering multiple fields ranging from engineering to medical [1] [2] [3] [4] [5]. These materials are not simply physical mixtures. They can be broadly defined as nanocomposites with organic and inorganic components, intimately mixed. Specifically, organic-inorganic nanocomposite is a heterogeneous system where (in most cases) inorganic component domains have a dimensions ranging from nanometers to several micrometers. The conventional route to prepare organic-inorganic nanocomposites corresponds to very convenient sol-gel chemistry, the use of bridged and polyfunctional precursors and hydrothermal synthesis. The more complicated approaches use self-assembly, templated growth by organic surfactants or integrative synthesis routes [6]. The most commonly used inorganic components in organic-inorganic nanocomposites are metal nanoparticles. A great amount of different methods for synthesis of metal nanoparticles have been developed including chemical, photochemical and thermal [7] [8] [9] [10]. Specifically, the photo-induced synthesis strategy has attracted much attention as it is versatile and convenient process with distinguishing advantages such as space-selective fabrication [11]. These nanoparticle synthesis strategies can be categorized into two distinct approaches - photophysical (top down) and photochemical (bottom up) ones. In particular, the direct photoreduction has been established as an important technique for nanoparticle synthesis, where direct excitation of metal source is employed for formation of metal nanoparticles. This technique has an advantage of being free from reducing agents and is widely employed in the various mediums including polymer coatings and thin films. Commonly, polymeric materials including polystyrene, poly(methyl methacrylate), poly(ethylene terephthalate, poly(vinyl chloride), polycarbonate and polyvinylpyrrolidone serve as organic matrices for incorporation of metal nanoparticles [12] [13] [14] [15] [16] [17]. In contrast, there has been little exploration into the use of crosslinkable silicone compositions to incorporate metal nanoparticles. Conventional crosslinkable silicone materials are cured by thermal treatment at specific temperatures. These silicones usually have reactive functional groups attached to the siloxane end chain acting as a catalyst in the curing process. It is known, that platinum catalysts are the most widely used in silicones industry. Highly active platinum catalysts rely on a hydrosilylation reaction, in which a Si-H bond is added across the unsaturated C=C double bond of an olefin, resulting in the formation of a Si-C bond [18]. Another class of crosslinkable silicone materials are cured by UV-radiation or electron beam (e-beam). These silicones also have specific functional modifications. Specifically, UV-cured acrylate functionalized silicones are very promising polymeric materials offering a great versatility with regard to cure-speed and coatability, which make them ideally suited for a variety of applications, in particular for coatings [19]. Additionally, as UV curing is performed at room temperature it allows working with heat-sensitive substrates. These silicone systems consist of either acrylate end-capped linear polydimethylsiloxanes or of silicone chains containing acrylate groups. Traditionally, UV-curing of acrylate functionalized silicones mainly was performed under inert conditions (e.g. N₂ or CO₂ gases). This was done because of the strong inhibitory effect of atmospheric oxygen on the free radical polymerization of acrylate functionalized silicones [20]. The incorporation of tertiary amines or phosphine groups has overcome this inconvenience, thus allowing the UV curing to be performed at atmospheric conditions [21].
Recently, silver nanoparticles containing polyurethane based nanocomposite coatings were proved to be effective against Gram-negative *Escherichia coli* and Gram-positive *Staphylococcus aureus* [22], which holds a great promise for potential medical and biomedical applications. R.D. Toker et. al prepared organic-inorganic nanocomposite coating from starting components: organic formulation composed of N-vinylpyrollidone (NVP) (20 wt.%), Laromer® PE 56F (22 wt.%), photomer® 4149F (2 wt.%), photomer® 5429F (1 wt.%), methoxy vinyl silane (2 wt.%), Irgacure 184 (1.5 wt.%), BYK331 (0.02 g) and urethane acrylate based oligomer (UA) (50 wt.%) (component A), a mixture of trimethoxysilane end-capped bis[(4-β-hydroxyethoxy)phenyl]methyl phosphine oxide urethane (MSTPOU), methacryloxypropyltrimethoxysilane, ethanol, distilled water and p-toluene sulfonic acid (component B) and chemically synthesized Ag nanoparticle solution from AgNO₃ source (component C).

However, the method proposed R.D. Toker et. al is highly economically inefficient due to multiple subsequent technological and operational procedures and a vast number of materials required for fabrication of related nanocomposite. Accordingly, there exists a need for efficient, cost-effective method for production of organic-inorganic nanocomposite with a good antibacterial activity.

### Summary of the Invention

The present invention was made throughout the years of scientific work with different silicone and nanoparticle compositions, as well as prior art described above subject to analysis. The object of the present invention is to provide a novel organic-inorganic hybrid formulation and synthesis method of nanocomposite where cross-linking of organic phase is obtained simultaneously with formation of silver nanoparticles by UV-radiation, thus obtaining efficient, easy and fast process for fabrication of nanocomposite with antibacterial activity. Additionally, to provide organic-inorganic hybrid formulation containing a reduced number of materials for nanocomposite fabrication, thus obtaining cost-effectiveness.

According to an aspect of the present invention, a method of fabricating silver nanoparticles containing aliphatic silicone acrylate based UV-cured organic-inorganic composite coating with antibacterial activity, the method includes dissolving a calculated amount of silver perchlorate salt in acrylic acid (Component A), adding appropriate amount of photoinitiator mixture of phosphine oxide, bis(2,6-dimethoxybenzoyl)(2,4,4-trimethylpentyl)-(9Cl) and 1-propanone,2-hydroxy-2-methyl-1-phenyl- (Irgacure 1700) (Component B) into the solution of Component A and mixing until a homogeneous solution is obtained (Component AB), mixing the Component AB with appropriate amount of aliphatic silicone acrylate based UV-curable polymer (CN9800) (Component C) until a homogeneous suspension is formed (Component ABC), forming a thin coating from the Component ABC, exposing the thin coating comprising of Component ABC to UV radiation for estimated time in order to cross-link the organic phase and at the same time to reduce the silver salt to silver nanoparticles.

The present inventors found organic-inorganic hybrid formulation where the number of components required to produce nanocomposite material is highly reduced in contrast to previously reported. Importantly, the present inventors found that cross-linking of aliphatic silicone acrylate based organic phase is obtained simultaneously with photochemical formation of silver nanoparticles via direct photoreduction of silver salt by UV-radiation. The method disclosed herein is very simple, fast and reliable.
Further features and aspects of the present invention will become apparent from the description of exemplary embodiments with reference to the attached drawings.

### Brief Description of the Drawings

**Fig. 1** is an Gaussian function fitted to the plasmon resonance peak appearing at the wavelength of 416 nm in UV-visible light spectra (337-840 nm) of silver nanoparticles containing aliphatic silicone acrylate based UV-cured organic-inorganic composite coating.
**Fig. 2** is an X-ray diffraction pattern of silver nanoparticles containing aliphatic silicone acrylate based UV-cured organic-inorganic composite coating with crystallographic plane orientations of silver phase indicated.
**Fig. 3** is an image of 5 µl water droplet on aliphatic silicone acrylate based UV-cured coating (A) and silver nanoparticles containing aliphatic silicone acrylate based UV-cured organic-inorganic composite coating (B) with a resulting stable static contact angle values of 94 ± 1° and 85 ± 1°, respectively.
**Fig. 4** is an illustration of antibacterial activity of aliphatic silicone acrylate based UV-curable organic-inorganic composite coating samples having a different concentration of silver nanoparticles, against E. coli and S. aureus bacteria.

### Detailed Description of the Invention

Described herein is the method of fabricating silver nanoparticles containing aliphatic silicone acrylate based UV-cured organic-inorganic composite coating with antibacterial activity. The materials and methods disclosed herein may be used in the mass production.

It has been discovered that using specific organic-inorganic hybrid formulation the cross-linking of organic phase is obtained simultaneously with formation of nanoparticles resulting from silver salt reduction process by UV-radiation. The organic-inorganic hybrid formulation contains aliphatic silicone acrylate based UV-curable polymer, silver salt, UV photoinitiator and organic solvent. The resultant silver nanoparticles containing aliphatic silicone acrylate based UV-cured organic-inorganic composite coating may be used for potential applications covering multiple fields ranging from engineering to medical. Notable applications include antibacterial coatings for medical devices, food package and industrial pipes.

### Examples

Hereinafter, the present invention is described in more detail and specifically with reference to the Examples, which however are not intended to limit the present invention.

### Example 1: fabrication of aliphatic silicone acrylate based UV-cured organic-inorganic composite coating having an estimated silver nanoparticle concentration of 0.5 %.

0.05 g of silver perchlorate salt was dissolved in 1.00 ml of acrylic acid (Component A). 0.10 g of photoinitiator mixture of phosphine oxide, bis(2,6-dimethoxybenzoyl)(2,4,4-trimethylpentyl)-(9Cl) (10.0 - 30.0 wt%) and 1-propanone,2-hydroxy-2-methyl-1-phenyl- (60 - 90 wt%) (Irgacure 1700) (Component B) was added into the solution of Component A and mixed at ambient temperature until a homogeneous solution was obtained (Component AB). The Component AB was mixed with 4.00 g of aliphatic silicone acrylate based UV-curable polymer (CN9800) (Component C) at ambient temperature until a homogeneous suspension was formed (Component ABC). A thin coating having a thickness of 1 mm was formed. Afterwards, the thin coating comprising of Component ABC was exposed to UV radiation (1 kW, duration 60 s, distance from sample 15 cm) for 120 s in order to cross-link the organic phase and at the same time to reduce the silver salt to silver nanoparticles.

### Example 2: fabrication of aliphatic silicone acrylate based UV-cured organic-inorganic composite coating having an estimated silver nanoparticle concentration of 1.0 %.

0.10 g of silver perchlorate salt was dissolved in 1.00 ml of acrylic acid (Component A). 0.20 g of photoinitiator mixture of phosphine oxide, bis(2,6-dimethoxybenzoyl)(2,4,4-trimethylpentyl)-(9Cl) (10.0 - 30.0 wt%) and 1-propanone,2-hydroxy-2-methyl-1-phenyl- (60 - 90 wt%) (Irgacure 1700) (Component B) was added into the solution of Component A and mixed at ambient temperature until a homogeneous solution was obtained (Component AB). The Component AB was mixed with 4.00 g of aliphatic silicone acrylate based UV-curable polymer (CN9800) (Component C) at ambient temperature until a homogeneous suspension was formed (Component ABC). A thin coating (thickness 1 mm) was formed. Afterwards, the thin coating comprising of Component ABC was exposed to UV radiation (1 kW, duration 60 s, distance from sample 15 cm) for 120 s in order to cross-link the organic phase and at the same time to reduce the silver salt to silver nanoparticles.

### Example 3: fabrication of aliphatic silicone acrylate based UV-cured organic-inorganic composite coating having an estimated silver nanoparticle concentration of 1.43 %.

0.15 g of silver perchlorate salt was dissolved in 1.00 ml of acrylic acid (Component A). 0.30 g of photoinitiator mixture of phosphine oxide, bis(2,6-dimethoxybenzoyl)(2,4,4-trimethylpentyl)-(9Cl) (10.0 - 30.0 wt%) and 1-propanone,2-hydroxy-2-methyl-1-phenyl- (60 - 90 wt%) (Irgacure 1700) (Component B) was added into the solution of Component A and mixed at ambient temperature until a homogeneous solution was obtained (Component AB). The Component AB was mixed with 4.00 g of aliphatic silicone acrylate based UV-curable polymer (CN9800) (Component C) at ambient temperature until a homogeneous suspension was formed (Component ABC). A thin coating (thickness 1 mm) was formed. Afterwards, the thin coating comprising of Component ABC was exposed to UV radiation (1 kW, duration 60 s, distance from sample 15 cm) for 120 s in order to cross-link the organic phase and at the same time to reduce the silver salt to silver nanoparticles.
The accompanying drawings, which are incorporated in and constitute a part of the specification, illustrate embodiments of the invention and, together with the description, serve to explain the principles of the invention.

### Example 4: fabrication of aliphatic silicone acrylate based UV-cured organic-inorganic composite coating having an estimated silver nanoparticle concentration of 1.43 %.

0.15 g of silver perchlorate salt was dissolved in 1.00 ml of acrylic acid (Component A). 0.30 g of photoinitiator mixture of phosphine oxide, bis(2,6-dimethoxybenzoyl)(2,4,4-trimethylpentyl)-(9Cl) (10.0 - 30.0 wt%) and 1-propanone,2-hydroxy-2-methyl-1-phenyl- (60 - 90 wt%) (Irgacure 1700) (Component B) was added into the solution of Component A and mixed at ambient temperature until a homogeneous solution was obtained (Component AB). The Component AB was mixed with 4.00 g of aliphatic silicone acrylate based UV-curable polymer (CN9800) (Component C) at temperature of 80 °C (Component ABC). A thin coating (thickness 1 mm) was formed. Afterwards, the thin coating comprising of Component ABC was exposed to UV radiation (1 kW, duration 60 s, distance from sample 15 cm) for 120 s in order to cross-link the organic phase and at the same time to reduce the silver salt to silver nanoparticles.
The accompanying drawings, which are incorporated in and constitute a part of the specification, illustrate embodiments of the invention and, together with the description, serve to explain the principles of the invention.

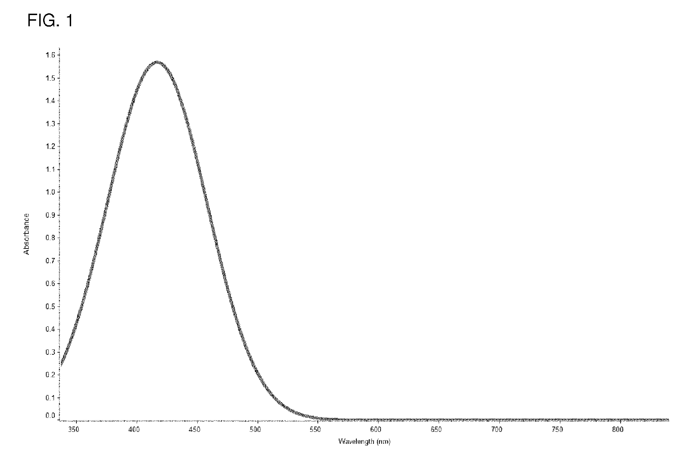

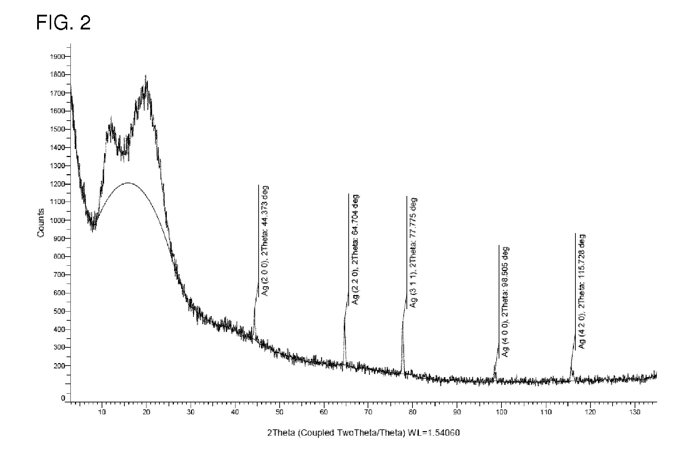

The information about size of silver nanoparticles was obtained by analyzing the spectral properties of silver nanoparticles embedded in a free standing aliphatic silicone acrylate based UV-cured organic-inorganic composite coating (prepared using Example 2) using an optical spectrometer Avantes that is composed of a deuterium halogen light source (AvaLight DHc) and spectrometer (Avaspec-2048). The analysis was performed in the wavelength region from 337 nm to 840 nm. The spectral response of silver nanoparticles is shown in Fig. 1, presented as a Gaussian function fitted to the plasmon resonance peak appearing at the wavelength of 416 nm in UV-visible light spectra. The peak wavelength, yield a unique spectral fingerprint for a plasmonic nanoparticles with a size corresponding to 20 ±5 nm. The crystallographic nature of the silver nanoparticles was determined using X-ray diffractometer D8 Discover (Bruker AXS GmbH) with parallel beam focusing geometry of Cu Kαᵢ (*λ*=0.154 nm) radiation. Processing of the resultant diffractograms was performed with DIFFRAC.EVA software. Phase identification and interpretation involved matching the diffraction pattern of free standing silver nanoparticles containing aliphatic silicone acrylate based UV-cured organic-inorganic composite coating (prepared using Example 2) to patterns of single-phase reference materials. Reference patterns from the Joint Committee on Powder Diffraction Standards-International Centre for Diffraction Data (JCPDS-ICDD) database were used. Fig. 2 shows an X-ray diffraction pattern of silver nanoparticles containing aliphatic silicone acrylate based UV-cured organic-inorganic composite coating. After reduction process, silver nanoparticles crystallized to cubic crystal structure (PDF, 04-016-5006) with space group Fm-3m (group number= 225) and the (2 0 0), (2 2 0), (3 1 1), (4 0 0) and (4 2 0) crystallographic plane orientations observable (Fig. 2).

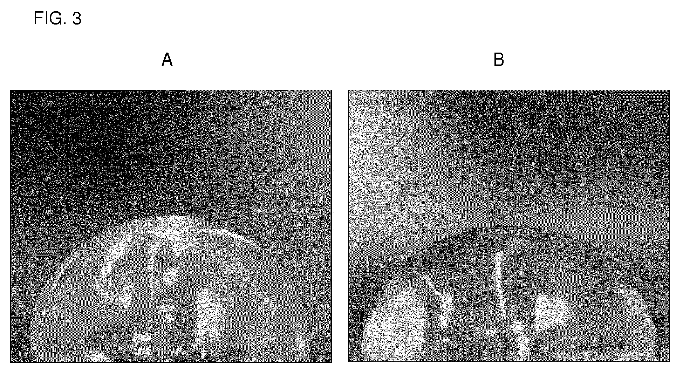

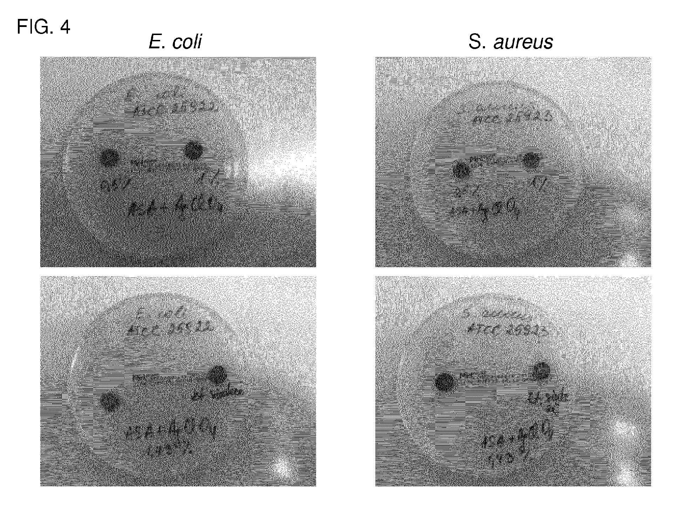

Surface wetting properties of silver nanoparticles containing aliphatic silicone acrylate based UV-cured organic-inorganic composite coating (prepared using Example 2) were determined using contact angle measurements. The measurements were performed at room temperature (23 °C) using sessile drop method. One droplet of deionized water (∼5 µl) was deposited on the sample surface. Optical images of the droplet were obtained and contact angle was measured using a method based on B-spline snakes (active contours). Fig. 3 shows a characteristic image of 5 µl water droplet on aliphatic silicone acrylate based UV-cured coating and silver nanoparticles containing aliphatic silicone acrylate based UV-cured organic-inorganic composite coating. The static contact angle on aliphatic silicone acrylate coating was determined to be 94 ± 1°, indicating that nanocomposite is hydrophobic, while after modification with silver nanoparticles contact angle decreased down to 85 ± 1°.
The antibacterial activities of the silver nanoparticles containing aliphatic silicone acrylate based UV-cured organic-inorganic composite coatings were determined using the disc diffusion method. The bacteria, *Escherichia coli* (E. coli) (ATCC 25922) and *Staphylococcus aureus* (S. aureus) (ATCC 25923), were chosen for the testing, as E. coli and S. aureus are the most commonly found bacteria. E. coli and S. aureus were cultured in Tryptone Soya Broth (TSB) solution (30 g/L). Bacteria were first cultured in a flask with 30 mL of TSB. The incubation was performed at 37 °C and oscillated at a frequency of 150 rpm for 24 h to obtain the overnight phase of the bacteria. Following that, about 1 mL amount of the E. coli and S. aureus were pipetted from the overnight phase into another flask with freshly prepared 30 mL of TSB, respectively. Bacteria were grown at 37 °C in the incubator shaker for another 5 h to obtain the bacteria with higher activity and more viable than in other growth phases. Antibacterial tests were carried out for aliphatic silicone acrylate based UV-cured organic-inorganic composite coatings having different concentration of silver nanoparticles. A commercially available Mueller Hinton Agar plates were used in antibacterial testing. The stationary phase bacterium was diluted into a concentration at about 10³ CFU/mL with NaCl solution (0.85%). Diluted suspensions (0.1 mL) of E. coli and S. aureus were transferred and spread onto the solid surface of the plate. In the inhibition experiment, the free standing organic-inorganic composite coating samples were placed on the E. coli and S. aureus agar plates and incubated at 37 °C for 24 h. Fig. 4 illustrates the antibacterial activity of the silver nanoparticles containing aliphatic silicone acrylate based UV-curable organic-inorganic composite coatings. The concentration of silver nano particles in composite coating is varied from 0.5 to 1.43 % (Example 1-4). In all cases, the antibacterial effect is clearly observed.

## Claims

1. A method of fabricating silver nanoparticles containing aliphatic silicone acrylate based UV-cured organic-inorganic composite coating with antibacterial activity, the method comprising:
- dissolving a calculated amount of silver perchlorate salt in acrylic acid (Component A);
- adding appropriate amount of photoinitiator mixture of phosphine oxide, bis(2,6-dimethoxybenzoyl)(2,4,4-trimethylpentyl)-(9Cl) and 1-propanone,2-hydroxy-2-methyl-1-phenyl- (Irgacure 1700) (Component B) into the solution of Component A and mixing until a homogeneous solution is obtained (Component AB);
- mixing the Component AB with appropriate amount of aliphatic silicone acrylate based UV-curable polymer (CN9800) (Component C) until a homogeneous suspension is formed (Component ABC);
- forming a thin coating from the Component ABC;
- exposing the thin coating comprising of Component ABC to UV radiation for estimated time in order to cross-link the organic phase and at the same time to reduce the silver salt to silver nanoparticles.

2. The organic-inorganic hybrid formulation contains aliphatic silicone acrylate based UV-curable polymer, silver salt, UV photoinitiator and organic solvent.
